Europäisches Patentamt

European Patent Office

Office européen des brevets

(19)

(11) Publication number: **0 307 525**
**A1**

## EUROPEAN PATENT APPLICATION

(21) Application number: **87308308.3**

(22) Date of filing: **18.09.87**

(51) Int. Cl.⁴: **A61K 9/06** , **A61F 9/00**

Claim 011 is deemed to be abandoned due to non-payment of the claims fee (Rule 31 (2) EPC).

(43) Date of publication of application:
**22.03.89 Bulletin 89/12**

(84) Designated Contracting States:
**DE FR GB IT SE**

(71) Applicant: York, Kenneth K.
**2300 North Edgemont Street**
**Los Angeles California 90027(US)**

(72) Inventor: York, Kenneth K.
**2300 North Edgemont Street**
**Los Angeles California 90027(US)**

(74) Representative: Marlow, Nicholas Simon et al
**Reddie & Grose 16, Theobalds Road**
**London WC1X 8PL(GB)**

(54) Biocompatible intraocular lightscreening compositions.

(57) Light-absorbing, ocularly biocompatible compositions that can be easily eliminated from the eye include at least one biocompatible, light-absorbing substance in an amount sufficient to absorb substantially all ultraviolet light, substantially all visible light of predetermined wavelengths, or both. The composition is placed intraocularly, in the anterior chamber, over the pupil, in the posterior chamber or in the capsular bag of an eye, as during an ophthalmic procedure. Where the composition includes a viscous, biocompatible, space-maintaining substance such as sodium hyaluronate, chondroitin sulfate or polyacrylamide, the composition also protects the endothelium, maintains the space within the eye, moves tissue, allows focal application of the light-absorbing substance, and permits rapid reversal of pupillary occlusion.

EP 0 307 525 A1

## BIOCOMPATIBLE INTRAOCULAR LIGHT-SCREENING COMPOSITIONS

This invention relates to biocompatible, light-absorbing compositions such as are used intraocularly to protect light-sensitive ocular tissues from photic damage during therapeutic and diagnostic ophthalmic procedures.

According to the invention there is provided a light-absorbing, biocompatible composition adapted to absorb substantially all ultraviolet light, substantially all visible light of predetermined wavelengths or both, when the substance is placed intraocularly over the pupil, in the posterior chamber or in the capsular bag of an eye.

These compositions absorb substantially all ultraviolet (UV) and/or visible light (especially short wavelength visible light, i.e., light having wavelengths in the range of about 400 to 500 nm, i.e., blue) when placed intraocularly in the anterior chamber of an eye over the pupil, in the posterior chamber or in the capsular bag. These compositions are eliminated from the eye over a relatively short period of time after application, varying from hours to days, without substantial damage to such tissue. The biocompatible, light-absorbing compositions may include a chromophore, chromogen, or a dye such as Fluorescein Sodium (Spiro [isobenzofuran-1(3H), 9'-(9H)xanthene]-3-one, 3'6'-dihyodroxy, disodium salt), which absorbs strongly in the blue portion of the spectrum or fine pigment particles, autologous packed red blood cells or liposomes containing a light-absorbing substance which will form a suspension. These biocompatible substances, in sufficient concentration, absorb substantially all ultraviolet and/or visible light, especially short wavelength visible light (blue), directed at them, and can be injected into the anterior chamber, posterior chamber or capsular bag of an eye to protect the lens and posterior segment structures.

In preferred embodiments, this invention relates to a composition including at least one biocompatible, UV light-absorbing and/or visible light-absorbing substance, such as Fluorescein Sodium, and a carrier, preferably a viscous, biocompatible, space-maintaining gel. This preferred embodiment concentrates the light-screening substance, delays its dispersion and permits focal application.

Contact with the corneal endothelium is avoided, thus minimizing the risk of thermal and photosensitization damage to these critical cells. Suitable biocompatible, viscous, space-maintaining gels include, but are not limited to, chondroitin sulfate, sodium hyaluronate and polyacrylamide.

These compositions can be applied intraocularly over the miotic pupil of a supine patient to block light during an ophthalmic procedure. After the procedure, when the patient assumes an erect position, the composition, being denser than water, will fall into the inferior angle of the eye, clearing the visual axis, and allowing the patient to see. The composition will be eliminated from the inferior angle of the eye without any deleterious effects through natural filtration means.

In preferred embodiments comprising at least one biocompatible light-absorbing substance and at least one carrier gel, the composition can vary, depending upon the nature of the light-absorbing substance and of the gel. For Fluorescein Sodium, a satisfactory mixture is one part 25% Fluorescein Sodium and nine parts 10% sodium hyaluronate, but a wide range of concentrations is feasible. The light-absorbing substances and the carriers for these substances are, in preferred embodiments, mixed with one another under sterile conditions in the desired ratios and for a time sufficient to disperse the light-absorbing substances uniformly in the carriers. These substances may be combined as mixtures, but preferably should be chemically bound.

Fluorescein Sodium is a particularly desirable light-absorbing substance because of its ocular biocompatibility, its long history of safe use in retinal angiography, and its capacity to strongly absorb light with wavelengths in the range of about 400 to about 500 nm. Moreover, Fluorescein Sodium is not a strong photo-sensitizer. Fluorescein Sodium does not absorb strongly in the red portion of the spectrum, and would permit viewing of a red laser or light for fixation.

The methods of this invention comprise forming an opening in the cornea or limbus of an eye of sufficient size to permit an ophthalmic cannula to pass through the opening, and then injecting the light-absorbing composition over the pupil of an eye before exposing the lens or retina of the eye to therapeutic or diagnostic ultraviolet light and/or visible light. Alternatively, the capsular bag or posterior chamber may be filled with an intraocular light-absorbing composition to facilitate intraocular lens implantation and provide protection from light-induced retinal damage during surgery. The quantity of the light-absorbing composition placed over the lens can vary, but, in practice, need not exceed the amount necessary to occlude the pupil. The quantity of the composition and the concentration of the light-absorbing substance should be sufficient to prevent ultraviolet light and/or visible light from reaching the lens, retina, or both, during a therapeutic or diagnostic ophthalmic procedure utilizing such light. Even intraocular lenses containing UV-absorbing compounds still transmit blue light, which has been shown to cause retinal damage.

The light-absorbing compositions in this invention are of particular importance in preventing or at least minimizing retinal damage in the form of cystoid macular edema (CME) and blue light photoretinitis (photo-chemical damage). CME and blue light photoretinitis are significant causes of permanent visual loss after cataract surgery. The intense light from coaxial illumination systems on operating microscopes during cataract surgery can cause retinal damage. Intraocular lenses that are implanted during most cataract operations exacerbate the problem by focusing and concentrating light on the macula, the most sensitive and visually important part of the retina. Wavelengths from about 290 nm to about 1,400 nm are transmitted through the cornea of the mammalian eye to the lens and retina. Ultraviolet light has more energy and causes more damage than the longer visible wavelengths. Similarly, the retina is much more susceptible to damage from blue light than infrared light. Three orders of magnitude more power is required to produce a minimal retinal lesion at 1,064 nm than at 441 nm (blue light).

Recognizing the hazard of short wavelength light, efforts to prevent photic damage have included the use of UV-absorbing microscope filters, eclipse microscopic filters, UV-absorbing intraocular lenses, occluders placed on the cornea, and anti-inflammatory drugs. None of these methods has been entirely successful. By contrast, the light-absorbing substances of this invention can be placed over the pupil of an eye prior to certain anterior segment laser procedures such as photokeratomileusis, or in the capsular bag or posterior chamber prior to intraocular lens implantation, or over the intraocular lens after implantation. These light-absorbing substances will protect the crystalline lens, vitreous and retina from unintentional photic damage from certain visible wavelengths and/or ultraviolet light.

Many ophthalmic surgeons today use a viscoelastic preparation in every cataract extraction/lens implant that they perform to protect the corneal endothelium, maintain space and move tissue. The mucopolysaccharides, sodium hyaluronate and chondroitin sulfate absorb in the infrared spectrum, but not sufficiently in the critical long UV and short visible light spectra. The preferred embodiment of this invention can perform all the functions of conventional viscoelastic preparations and, in addition, protect the crystalline lens and retina from photic damage.

## Claims

1. A light-absorbing, biocompatible composition adapted to absorb substantially all ultraviolet light, substantially all visible light of predetermined wavelengths or both, when the substance is placed intraocularly over the pupil, in the posterior chamber or in the capsular bag of an eye.

2. A composition according to claim 1 including a chromophore, chromogen or dye and a carrier.

3. A composition according to claim 2, in which the carrier comprises at least one viscous, space-maintaining, biocompatible substance.

4. A composition according to any preceding claim in which the light-absorbing substance is present in an amount sufficient to absorb substantially all ultraviolet and visible light.

5. A composition according to any preceding claim in which the light-absorbing substance is Fluorescein Sodium.

6. A method for protecting ocular tissues such as the lens, vitreous and retina of an eye from damage caused by ultraviolet light, visible light of predetermined wavelengths, or both, comprising placing intraocularly over the pupil, in the posterior chamber, or in the capsular bag of an eye, in an amount sufficient to prevent substantially all ultraviolet light, substantially all visible light, or both, from reaching such tissues during ophthalmic procedures, a light-absorbing composition comprising a carrier and at least one biocompatible, light-absorbing substance in an amount sufficient to absorb substantially all of said ultraviolet light, substantially all of said visible light of predetermined wavelengths, or both.

7. The method of claim 6 wherein said carrier comprises at least one viscous, space-maintaining, biocompatible substance.

8. The method of claim 6 wherein said carrier comprises at least one viscous, space-maintaining, biocompatible substance and wherein the biocompatible, light-absorbing substance is a chromophore, a chromogen, a dye or a suspension.

9. The method of claim 6 wherein said viscous, space-maintaining, biocompatible carrier is a mucopolysaccharide, and wherein the biocompatible, light-absorbing substance is Fluorescein Sodium.

10. A method for protecting the lens and retina of an eye from damage caused by ultraviolet light and/or visible light of predetermined wavelengths, or both, comprising placing over such tissues, in an amount sufficient to prevent substantially all ultraviolet light, substantially all visible light, or both, from reaching such tissues during a therapeutic or diagnostic ophthalmic procedure, a light-absorbing, biocompatible substance in an amount sufficient to absorb substantially all of said ultraviolet light, substantially all of said visible light of predetermined wavelengths, or both, that would otherwise impinge on such tissues.

11. The method of claim 10 wherein the light-absorbing substance is Fluorescein Sodium.

European Patent Office

**PARTIAL EUROPEAN SEARCH REPORT**
which under Rule 45 of the European Patent Convention
shall be considered, for the purposes of subsequent
proceedings, as the European search report

Application number

EP 87 30 8308

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | WO - A - 86 02 548 (PHARMACIA) <br><br> * Page 1, lines 1-32; claims 1-4 * | 1-5 | A 61 K 9/06 <br> A 61 F 9/00 |
| A | CHEMICAL ABSTRACTS, vol. 94, no. 8, 23rd February 1981, page 364, abstract 52832h Columbus, Ohio, US; V.A. NURITDINOV: "Polyacrylamide as a basis for medicinal eye films" & VESTN. OFTAL'MOL. 1980, (5), 59-60 <br><br> * Abstract * | 1-5 | |
| A | CHEMICAL ABSTRACTS, vol. 105, no. 20, 17th November 1986, page 377, abstract 178382a Columbus, Ohio, US; H.M. CLAYMAN: "Ultraviolet-absorbing chromophores: chemical and ultra-violet transmission characteristics" & CATARACT REFRACTIVE SURG. 1986, 12(5), 529-32 <br><br> * Abstract * | 1-5 | **TECHNICAL FIELDS SEARCHED (Int. Cl.4)** <br><br> A 61 K 9/06 <br> A 61 F 9/00 |

## INCOMPLETE SEARCH   -2-

The Search Division considers that the present European patent application does not comply with the provisions of the European Patent Convention to such an extent that it is not possible to carry out a meaningful search into the state of the art on the basis of some of the claims.

Claims searched completely:     1-5
Claims searched incompletely:
Claims not searched:     6-11
Reason for the limitation of the search:

Method for treatment of the human or animal body by surgery or therapy (see art. 52(4) of the European Patent Convention).

| Place of search | Date of completion of the search | Examiner |
|---|---|---|
| THE HAGUE | 09-05-1988 | PELTRE |

**PARTIAL EUROPEAN SEARCH REPORT**

| | DOCUMENTS CONSIDERED TO BE RELEVANT | | CLASSIFICATION OF THE APPLICATION (Int. Cl 4) |
|---|---|---|---|
| Category | Citation of document with indication, where appropriate, of relevant passages | Relevant to claim | |
| A | FR - A - 707 525 (A.M.HALPHEN) | | |
| | * Abstract; lines 65-76 * | 1 | |
| | -- | | |
| A | US - A - 4 328 803 (L.G. PAPE) | 1 | |
| | -- | | |
| A | FR - A - 2 073 437 (BURTON PARSONS) | | |
| | * Page 17, lines 1-4 * | 1 | |
| | -- | | |
| A | FR - A - 2 315 949 (BURTON PARSONS) | | |
| | * Page 14, lines 33-36 * | 1 | |

-------------------

**TECHNICAL FIELDS SEARCHED (Int. Cl.4)**

EPO Form 1505.3   06.78